# EUROPÄISCHE PATENTSCHRIFT

(11) **EP 1 161 437 B1**
(45) Veröffentlichungstag und Bekanntmachung des Hinweises auf die Patenterteilung: **06.11.2002**
(21) Anmeldenummer: 00910839.0
(22) Anmeldetag: 15.03.2000
(51) Int. Cl.: C07F 9/6568

(54) **PHOSPHABENZOLVERBINDUNGEN UND IHRE VERWENDUNG ALS LIGANDEN FÜR HYDROFORMYLIERUNGSKATALYSATOREN**
PHOSPHABENZENE COMPOUNDS AND THEIR USE AS LIGANDS FOR HYDROFORMYLATION CATALYSTS
COMPOSES DE PHOSPHABENZOL ET LEUR UTILISATION COMME LIGANDS DE CATALYSEURS D'HYDROFORMYLATION

(30) Priorität: 17.03.1999 DE 19911920
(43) Veröffentlichungstag der Anmeldung: 12.12.2001
(73) Patentinhaber: BASF AKTIENGESELLSCHAFT, 67056 Ludwigshafen (DE)
(72) Erfinder: PACIELLO, Rocco, D-67098 Bad Dürkheim (DE); MACKEWITZ, Thomas, D-68219 Mannheim (DE); RÖPER, Michael, D-67157 Wachenheim (DE); BREIT, Bernhard, D-69198 Schriesheim (DE)
(74) Vertreter: Isenbruck, Günter, Dr.
(86) Internationale Anmeldenummer: EP0002288
(87) Internationale Veröffentlichungsnummer: WO00055164

(56) Entgegenhaltungen:
- WO-A-97/46507
- DE-A- 1 618 668
- DE-A- 1 668 416

## Beschreibung

Die vorliegende Erfindung betrifft Phosphabenzolverbindungen und deren Verwendung in Komplexen von Übergangsmetallen der Nebengruppe VIII des Periodischen Systems der Elemente bei der Herstellung von Aldehyden durch Hydroformylierung von Olefinen mit CO/H₂ bei Temperaturen bis 200°C und Drücken bis 700 bar.

Die Hydroformylierung ist ein bekanntes, großtechnisch genutztes Verfahren zur Herstellung von Aldehyden aus Olefinen, Kohlenmonoxid und Wasserstoff. Wie in WO 97/46507 beschrieben, stellen Phosphabenzole aktive Cokatalysatoren für die Hydroformylierung von Olefinen dar. Es wird ein Verfahren zur Herstellung von Aldehyden durch Hydroformylierung von Olefinen mit CO/H₂ in Gegenwart von Komplexen beschrieben. die Phosphabenzolverbindungen als Liganden enthalten.

Die eingesetzten Phosphabenzolverbindungen wie 2,4,6-Triphenylphosphabenzol und 2,6-Bis(2-naphthyl)-4-phenylphosphabenzol, aber auch Phosphabenzole wie 2,3,5,6-Tetraphenylphosphabenzol oder 2,3,4,5,6-Pentaphenylphosphabenzol weisen jedoch den Nachteil auf, daß sie unter Hydroformylierungsbedingungen durch partielle oder vollständige Hydrierung des Phosphabenzolsystems und durch nachfolgende Additionsreaktionen abgebaut werden können (siehe Beispiele 11-14). Hierbei entstehen unter anderem sekundäre und tertiäre Phosphine, welche die Hydroformylierungsaktivität des Katalysatorsystems stark bremsen.

Ähnliche Phosphabenzolverbindungen sind in DE-A-19 621 967 und DE-A-16 68 416 beschrieben.

Aufgabe der vorliegenden Erfindung ist die Bereitstellung von Phosphabenzolliganden, die die Nachteile der bekannten Liganden vermeiden.

Die Aufgabe wird erfindungsgemäß gelöst durch Phosphabenzolverbindungen der allgemeinen Formel (I) 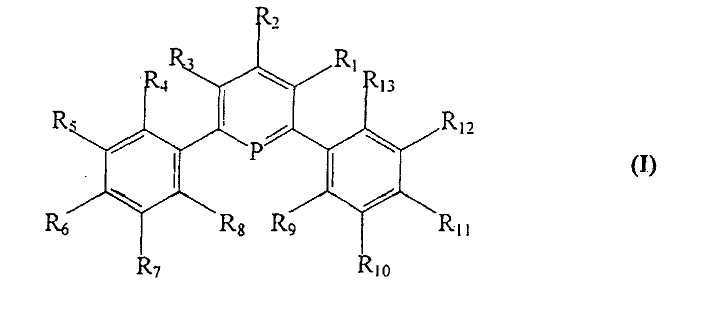 in der die Reste R¹ bis R¹³ unabhängig voneinander Wasserstoff, COOM, SO₃M, NR₃X, NR₂, OR, COOR oder SR (mit M = Wasserstoff, NH₄ oder Alkalimetall, X = Anion, R = Wasserstoff oder C₁₋₆-Alkyl), oder C₁₋₁₂-Alkyl, C₆₋₁₂-Aryl, C₇₋₁₂-Aralkyl, C₇₋₁₂-Alkaryl oder C₃₋₆-Heteroaromaten bedeuten, wobei die Alkyl-, Aryl-, Alkaryl- und Aralkylreste mit den davor genannten Resten substituiert sein können und zwei oder mehrere der Reste zu aliphatischen oder annelierten Ringen verbunden sein können, wobei mindestens einer der Reste R⁴ und R⁸ und mindestens einer der Reste R⁹ und R¹³ von Wasserstoff verschieden sind.

Vorzugsweise bedeuten mindestens einer der Reste R⁴ und R⁸ und mindestens einer der Reste R⁹ und R¹³ unabhängig voneinander C₁₋₁₂-Alkyl, C₆₋₁₂-Aryl, C₇₋₁₂-Aralkyl oder C₇₋₁₂-AIkaryl, oder R⁴ und R³ und/oder R¹³ und R¹ bilden einen C₂₋₄-Alkylenrest.

Besonders bevorzugt bedeuten mindestens einer der Reste R⁴ und R⁸ und mindestens einer der Reste R⁹ und R¹³ C₁₋₆-Alkyl, oder (R⁴ und R³) und (R¹³ und R¹) bilden jeweils einen C₂₋₃-Alkylenrest.

Vorzugsweise bedeutet R² einen Phenylrest, der durch 1 bis 5, vorzugsweise 1 bis 3, insbesondere 1 oder 2 C₁₋₆-Alkylreste substituiert sein kann.

Besonders bevorzugt bedeuten die Reste R¹ und R³ Wasserstoff, und jeweils maximal drei der Reste R⁴ bis R⁸ und R⁹ bis R¹³ sind vom Wasserstoff verschieden. Dabei liegen in den Resten R⁴ bis R⁸ und R⁹ bis R¹³ jeweils besonders bevorzugt maximal 6, insbesondere maximal 3 C-Atome vor. Insbesondere weisen die Phosphabenzolverbindungen der allgemeinen Formel (I) außer dem einen Phosphoratom keine von Kohlenstoff oder Wasserstoff verschiedenen Atome auf.

Bevorzugt liegen in den Verbindungen der allgemeinen Formel (I) neben dem Phosphabenzolkern 3 bis 5, insbesondere 3 weitere aromatische Kerne vor. Die Anzahl der Alkylreste in den Verbindungen der allgemeinen Formel (I) beträgt vorzugsweise 0 bei rein cyclischen Strukturen, ansonsten vorzugsweise 2 bis 7, insbesondere 2 bis 6. Bei den Alkylresten kann es sich um lineare oder verzweigte Alkylreste handeln. Vorzugsweise liegen nur lineare Alkylreste vor. Gleiches gilt für verbrückende Alkylengruppen entsprechend.

Beispielhaft seien folgende Phosphabenzole genannt:

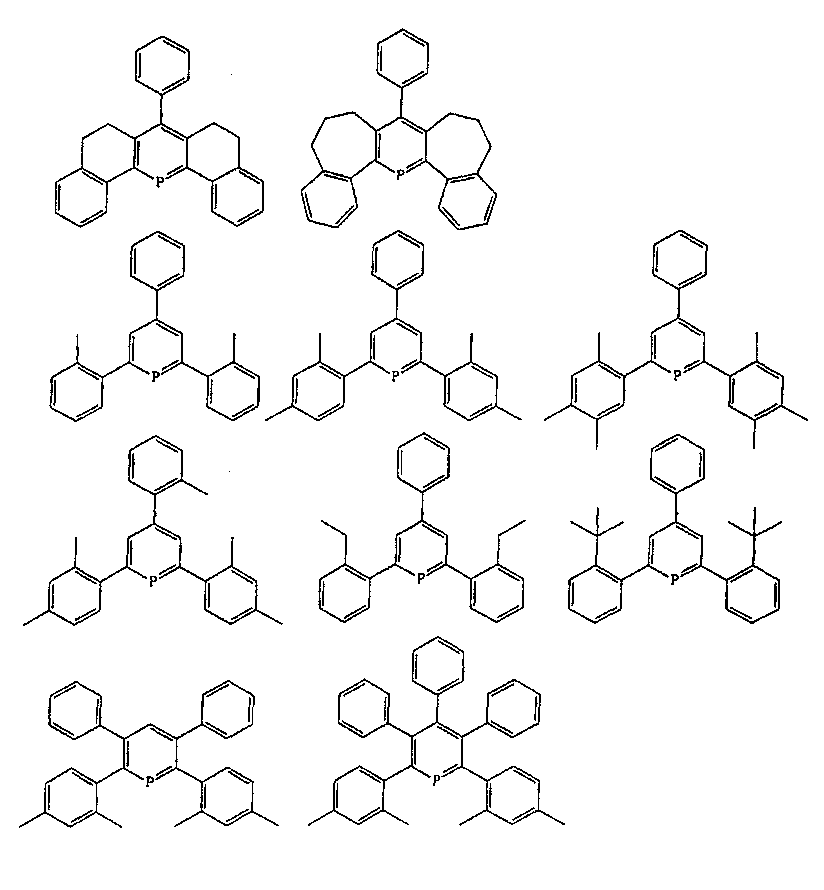

Es wurde gefunden, daß insbesondere die Einführung von 2-Alkylarylsubstituenten in 2- und 6-Position des Phosphabenzolsystems eine deutlich erhöhte Stabilität des Cokatalysators unter Hydroformylierungsbedingungen bewirkt, und die Katalysatorsysteme vergleichbare Aktivitäten wie entsprechende unsubstituierte Systeme liefern.

Der Abbau von Phosphabenzolverbindungen mit 2-Alkylaryl-substituenten in 2-und 6-Position des Phosphabenzolsystems unter Hydroformylierungsbedingungen ist deutlich verringert gegenüber analogen Systemen mit unsubstituierten Arylsubstituenten.

Das Prinzip der Herstellung der Phosphabenzole ist bekannt. Generelle Syntheseverfahren sind zu finden in G. Märkl in Multiple Bonds and Low Coordination in Phosphorus Chemistry (Hrsg. M. Regitz, O. J. Scherer), Thieme, Stuttgart, 1990, S. 220 bis 257 (und dort zitierte Literatur). Verfahren zur Herstellung von Phosphabenzolverbindungen aus Pyryliumsalzen durch Umsetzung mit Phosphin sind in WO 97/46507, DE-A-196 21 967 und der prioritätsälteren, nicht vorveröffentlichten DE-A-197 43 197 beschrieben.

Sie erfolgt vorzugsweise durch Umsetzung entsprechender Pyryliumsalze mit PH₃ in Gegenwart oder Abwesenheit einer katalytischen Menge Säure oder Base und in Anwesenheit oder Abwesenheit eines Löse- oder Verdünnungsmittels. Vorzugsweise werden die Pyryliumsalze mit PH₃ bei einer Temperatur oberhalb von 0°C zusammengebracht und bei einer Temperatur von 0°C bis 200°C und einem Druck von oberhalb 1 bar umgesetzt.

Es wurde erfindungsgemäß gefunden, daß Phosphabenzolverbindungen der vorstehenden allgemeinen Formel durch Umsetzung der entsprechenden Pyryliumsalze, d. h. Verbindungen, in denen in der allgemeinen Formel anstelle von Phosphor O⁺ mit entsprechendem Gegenion vorliegt, mit PH₃ erhältlich sind, wenn bestimmte Verfahrensbedingungen eingehalten werden. Die Pyryliumsalze sind dabei technisch erhältlich oder können einfach hergestellt werden. PH₃ ist technisch verfügbar.

Die Umsetzung erfolgt dabei vorzugsweise bei einem PH₃-Partialdruck im Bereich von 0,1 bis 100 bar, besonders bevorzugt von 5 bis 35 bar, insbesondere 20 bis 30 bar. Der Gesamtdruck im System hängt vom jeweiligen Lösungsmittel ab. Durch Aufpressen von PH₃ oder Inertgas kann der Gesamtdruck erhöht werden.

Vorzugsweise wird während der Umsetzung PH₃ in das Reaktionsgemisch eingeleitet, um den PH₃-Partialdruck im wesentlichen konstant zu halten. Diese Vorgehensweise erlaubt eine besonders wirtschaftliche und schnelle Umsetzung zu den gewünschten Phosphabenzolverbindungen. Dabei werden hohe Produktreinheiten und Umsätze erzielt. Das erfindungsgemäße Verfahren ist für eine Vielzahl von Produkten zuverlässig einsetzbar. Es kann kontinuierlich oder diskontinuierlich, vorzugsweise diskontinuierlich betrieben werden.
Bei einer besonders vorteilhaften Verfahrensvariante werden die Pyryliumsalze bei Umgebungstemperatur mit PH₃ zusammengebracht, und das so erhaltene Gemisch wird zur Umsetzung auf eine Temperatur im Bereich von 60 bis 140°C, bevorzugt 80 bis 130°C erhitzt.

Besonders bevorzugt beträgt die Temperatur bei der Umsetzung 100 bis 120°C. Die Umsetzung wird vorzugsweise in einem Autoklaven durchgeführt. Neben PH₃ kann zusätzlich ein Inertgas verwendet werden, mit dem der gewünschte Gesamtdruck eingestellt wird. Vorzugsweise wird jedoch nur PH₃ eingesetzt.

Die Umsetzung kann in Anwesenheit oder Abwesenheit eines Löse- oder Verdünnungsmittels durchgeführt werden. Vorzugsweise wird sie in Anwesenheit eines Löse- oder Verdünnungsmittels durchgeführt. Geeignete Löse- oder Verdünnungsmittel sind beispielsweise niederere aliphatische Alkohole, wie Methanol, Ethanol, n-Propanol, i-Propanol, n-Butanol, i-Butanol, tert.-Butanol oder Pentanol-Isomere, vorzugsweise Ethanol, Propanol oder Butanole, insbesondere n-Butanol.

Die Umsetzung kann in Gegenwart eines Säurekatalysators durchgeführt werden. Geeignete Säurekatalysatoren sind Mineralsäuren, wie HI, HCI, HBr. Insbesondere wird Bromwasserstoff in Essigsäure oder Essigsäureanhydrid als Säurekatalysator eingesetzt. Vorzugsweise wird ohne Säurekatalysator gearbeitet.

Nach der Umsetzung wird das Reaktionsgemisch vorzugsweise entspannt und gegebenenfalls mit einem Inertgas gespült, wobei die aus dem Reaktionsgemisch austretenden Gase zur Abtrennung von nicht umgesetztem PH₃ in flüssiger Form gekühlt und durch einen Abscheider geführt werden und das abgetrennte PH₃ in die Umsetzung zurückgeführt wird.

Besonders wirtschaftlich und ökologisch unbedenklich ist ein Verfahren, bei dem PH₃ in einen Reaktor eingeleitet wird, die Reaktion durchgeführt wird, und der Gasstrom über eine weitere Leitung durch einen Kühler beliebiger Bauart geleitet wird, in dem das PH₃ auskondensiert wird. In einem nachfolgenden Abscheider beliebiger Bauart wird das PH₃ sodann abgetrennt und beispielsweise mit Hilfe einer Pumpe in die Umsetzung zurückgeführt. Um ein besonders PH₃-armes Abgas zu erhalten, ist die Verwendung eines nachgeschalteten zweiten Kühlers und Abscheiders vorteilhaft. Um den Reaktorgasraum und die eingesetzten Apparaturen vollständig von PH₃ zu befreien, was wegen der Giftigkeit von PH₃ vorteilhaft ist, sollte eine Spülleitung für die Spülung mit einem Inertgas wie Stickstoff vorgesehen sein. Dabei sollte das Spülgas über die Kombination aus Kühler und Abscheider geleitet werden.

Die für die Umsetzung benötigte Zeit hängt von der Art des Pyryliumsalzes ab. Die Umsetzung wird je nach Pyryliumsalz vorzugsweise für einen Zeitraum von 1 bis 4 Stunden durchgeführt. Die Menge an eingesetztem Säurekatalysator beträgt, bezogen auf die Pyryliumsalze, vorzugsweise 0,01 bis 1 %, besonders bevorzugt 0,03 bis 0,1 %. Bei der Umsetzung mit einem Lösungsmittel hängt die Konzentration an PH₃ im Lösungsmittel vom PH₃-Partialdruck und der Art des Lösungsmittels ab; insbesondere bei kontinuierlicher Reaktionsführung sollte eine hohe Konzentration an PH₃ im Lösungsmittel beibehalten werden.

Zum Erreichen hoher Umsätze in kurzer Reaktionszeit wird vorzugsweise mit hohen PH₃-Drücken und kontinuierlichem Nachpressen von PH₃ gearbeitet.

Im erfindungsgemäßen Verfahren kann eine Vielzahl unterschiedlicher Pyryliumsalze eingesetzt werden. Das Verfahren ist allgemein nicht auf bestimmte Verbindungsklassen eingeschränkt. Beispielsweise können die Pyryliumsalze, Ferrate, Zinkate, Chloride, Borate, gegebenenfalls mit einem C₁₋₁₆-Alkylrest, Triflate, Trifluoracetate oder bevorzugt Tetrafluorborate, Perchlorate, Hydrogensulfate, Bromide, lodide oder Gemische davon sein. Vorzugsweise werden Tetrafluorborate eingesetzt. Der organische Rest der erfindungsgemäß eingesetzten Pyryliumsalze wird nachstehend anhand der daraus hergestellten Phosphabenzolverbindungen näher beschrieben.

Nach diesen Verfahren lassen sich auch die obigen Verbindungen herstellen. Noch nicht im einzelnen beschriebene Verbindungen werden analog erhalten.

Die Aktivität von Phosphanbenzolverbindungen mit 2-Alkylarylsubstituenten in 2- und 6-Position des Phosphabenzolsystems als Cokatalysator in der Hydroformylierung ist mit analogen Systemen, welche unsubstituierte Arylsubstituenten tragen, vergleichbar (siehe WO 97/02757 und Beispiele 6, 7, 9 und 10).

Die erfindungsgemäßen Verbindungen sind zur Herstellung von Komplexen mit Metallen der VIII. Nebengruppe des Periodensystems der Elemente verwendbar. Derartige Komplexe können als (Co)katalysatoren bei Hydroformylierungen von Olefinen mit CO/H₂ eingesetzt werden. Geeignete Umsetzungsbedingungen sind in der DE-A-196 21 967 und der WO 97/02757 beschrieben.

Die wirksamen Katalysatoren sind solche der Formel M(L)ₙ(CO)ₘ, in der M mindestens ein Zentralatom eines Elements der VIII. Nebengruppe des periodischen Systems der Elemente, L mindestens ein Ligand der Formel I, n und m jeweils mindestens 1 bis 3 und die Summe von n+m 2 bis 5 bedeuten und wobei noch weitere Reste wie Hydrido- oder Alkyl- oder Acylreste als Liganden enthalten sein können.

Der aktive Carbonylkomplex wird dabei in der Regel in situ, d. h. im Hydroformylierungsreaktor, aus einem Salz oder einer Verbindung des Metalls M, dem Liganden und Kohlenmonoxid gebildet; er kann aber auch getrennt hergestellt und als solcher eingesetzt werden.

Die Komplexkatalysatoren bestehen bevorzugt aus einem Zentralatom M, ausgewählt aus den Übergangsmetallen Cobalt, Ruthenium, Rhodium, Palladium oder Platin, insbesondere aber Cobalt und Rhodium, komplexiert mit Carbonylsowie Hydrido-, Alkyl- oder Acylresten sowie als Liganden die erfindungsgemäß zu verwendenden ein- oder mehrzähnigen bevorzugten Phosphabenzole. Werden die Komplexkatalysatoren in situ erzeugt, setzt man einfache Precursorkomplexe wie Rhodiumbiscarbonylacetylacetonat oder Rhodiumacetat in Gegenwart der entsprechenden Liganden den Reaktionsbedingungen aus, oder man versetzt Precursorkomplexe mit aktivierenden Zusätzen wie zum Beispiel Brönsted- oder Lewissäuren sowie Lewisbasen.

Zur in situ Bildung des Katalysators im Reaktionsgemisch setzt man den Ligand im molaren Verhältnis (gerechnet als Äquivalent Phosphor) zu Rhodium von 1 : 1 bis 1000 : 1 ein und verwendet zusätzlich ein inertes Lösungsmittel. Besonders bevorzugte Lösungsmittel sind die Aldehyde, die durch Umsetzung des jeweiligen Olefins entstehen, sowie die syntheseeigenen Hochsieder, die durch Folgereaktionen des jeweiligen Aldehyds im Hydroformylierungsverfahren entstehen. Bei durch geeignete Substituenten hydrophilisierten Liganden werden bevorzugt Wasser, Alkohole oder andere polare Lösungsmittel eingesetzt.

Die Zusammensetzung des im erfindungsgemäßen Hydroformylierungsverfahren eingesetzten Synthesegases CO/H₂ kann in weiten Bereichen variiert werden. Beispielsweise kann Synthesegas mit CO/H₂-Molverhältnissen von 5 : 95 bis 70 : 30 erfolgreich eingesetzt werden, bevorzugt wird Synthesegas mit CO/H₂-Verhältnissen von 40 : 60 bis 60 : 40, besonders bevorzugt wird ein CO/H₂-Verhältnis von etwa 1 :1 angewandt.

Die Hydroformylierungsreaktion mit CO und H₂ in Gegenwart des Katalysators wird bei einer Temperatur von 0 bis 200°C, vorzugsweise bei einer Temperatur zwischen 20 und 180°C, insbesondere 50 bis 150°C, durchgeführt. Für jedes Katalystorsystem wird jedoch zweckmäßig eine optimale Temperatur experimentell ermittelt. Der Reaktionsdruck kann je nach (Co)katalysator, d. h. Ligand, und Substrat in einem Bereich von Normaldruck, d.h. Atmosphärendruck, bis 700 bar, vorzugsweise bis 300 bar, schwanken, wobei man normalerweise Reaktionen in einem Bereich bis zu etwa 30 bar als Niederdruck-, in einem Bereich bis zu etwa 100 bar als Mitteldruck- und über 100 bar als Hochdruckreaktionen bezeichnet.

Dabei arbeitet man in der Regel mit dem homogen im Reaktionsmedium gelösten Katalysator, der vom Austrag der Hydroformylierungsreaktion abgetrennt und in die Hydroformylierungsstufe zurückgeführt wird.

Man erhält in der Regel nahezu ausschließlich die entsprechenden Aldehyde in ausgezeichneten Ausbeuten.

Als erfindungsgemäß zu hydroformylierende Olefine kommen α-Olefine oder interne Olefine oder interne, verzweigte Olefine in Betracht. Beispielsweise seien folgende Olefine genannt: Ethylen, Propen, 1-Buten, 1-Octen, C₅₋₂₀-α-Olefine, linare C₅₋₂₀ interne-Olefine, Buten-2; verzweigte, interne Octen-Gemische; verzweigte, interne Nonen-Gemische; verzweigte, interne Dodecen-Gemische, Cyclohexen, α-Pinen, Styrol, 4-Isobutylstyrol, 3-Pentensäuremethylester, 4-Pentensäuremethylester, Ölsäuremethylester, 3-Pentennitril, 4-Pentennitril, 2,7-Octadienol-1, 7-Octenal, Acrylsäuremethylester, Methacrylsäuremethylester, Acrylnitril, Vinylacetat, Vinylglykoldiacetat, Vinylmethylether, Polypropen, Polyisobutylen. Ebenfalls geeignete Substrate sind Di- oder Polyene mit isolierten oder konjugierten Doppelbindungen. Beispiele sind 1,3-Butadien, 1,5-Hexadien, Vinylcyclohexen, Dicyclopentadien, 1,5,9-Cyclooctatrien, Butadienhomo- und Copolymere, Polyisobuten.

Darüber hinaus wird die Hydroformylierungsreaktion in an sich bekannter Weise durchgeführt. Einzelheiten der Verfahrensführung sind Beller, et al., Journal of Molecular Catalysis A: 104 (1995) 17 - 85 und Falbe, Ed. New Syntheses with Carbon Monoxide, Springer, Berlin 1980, S. 55ff., zu entnehmen.

Die Erfindung wird nachstehend anhand von Beispielen näher erläutert:

### Beispiel 1

### Herstellung von 2,6-Bis(2,4-dimethylphenyl)-4-phenylpyryliumtetrafluoroborat

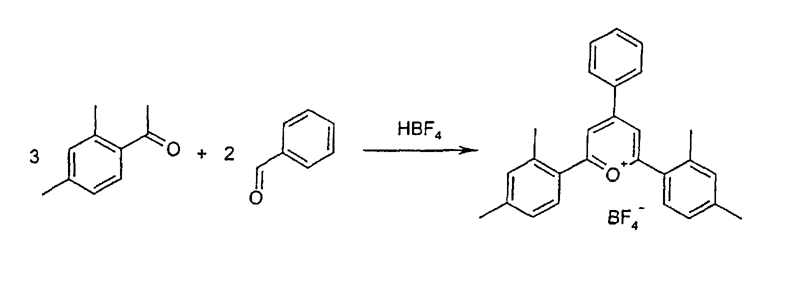

271 g (2.6 mol) Benzaldehyd und 542 g (3.7 mol) 2,4-Dimethylacetophenon wurden in 400 ml 1,2-Dichlorethan gelöst und auf 80 °C erwärmt. Hierzu tropfte man langsam unter Rühren 606 g (3.7 mol) einer 54 %ige etherische Tetrafluoroborsäure-Lösung. Anschließend rührte man bei dieser Temperatur weitere 4 h nach, und ließ dann auf Raumtemperatur abkühlen. Die flüchtigen Bestandteile der erhaltenen, tiefroten Lösung wurden im Hochvakuum unter leichtem Erwärmen abdestilliert. Der Rückstand wurde mit einem Toluol/Wasser-Gemisch (1:1) versetzt. Der ausgefallene, orangegelbe Feststoff wurde abfiltriert, mit Wasser und Toluol gewaschen und im Hochvakuum getrocknet. Zum Umkristallisieren wurde der Feststoff in Methanol suspendiert und anschließend solange mit Dichlormethan versetzt, bis eine klare Lösung vorlag. Unter leichtem Erwärmen wurde das Lösungsmittel im Hochvakuum abdestilliert, bis erneut Feststoff ausfiel. Das Produkt wurde abfiltriert, nacheinander mit Methanol und n-Pentan gewaschen und anschließend im Hochvakuum getrocknet. Ausbeute: 180 g (32 %) hellgelber Feststoff.

### Allgemeine Versuchsbeschreibung zur Herstellung von Phosphabenzolen

Alle folgenden Versuche (diskontinuierlich) wurden in einem 300 ml Autoklav (Material: HC) durchgeführt. Der Autoklav wurde mit Pyryliumsalz und einem geeigneten Lösungsmittel befüllt und mit 5 bar Stickstoff abgepresst. Der Gasraum wurde anschließend mit PH₃ gespült. Es wurden 5 bar PH₃ bei Raumtemperatur aufgepresst und mit PH₃ nachgepresst, bis der Druck bei 5 bar konstant blieb. Das Reaktionsgemisch wurde auf 110 °C erhitzt, und die Lösung wurde mit einem Begasungsrührer kräftig gerührt. Es wurde PH₃ bis zu einem Druck von 30 bar nachgepresst. Während der Reaktion wurde der Druck im Reaktor durch Nachpressen über einen Druckregler auf dem gewünschten Druckniveau gehalten. Nach einer Reaktionszeit von 4 h wurde der Autoklav abgekühlt, entspannt, mit Stickstoff unter Rühren gründlich ausgegast und ausgebaut. Der Autoklavenaustrag wurde dann wie nachstehend beschrieben aufgearbeitet.

### Beispiel 2

### Herstellung von 2,6-Bis(2,4-dimethylphenyl)-4-phenylphosphabenzol

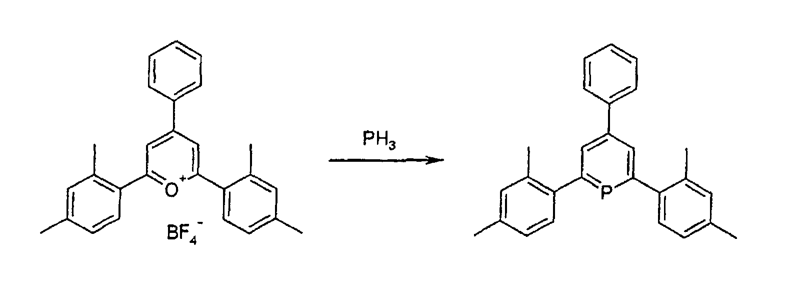

Es wurden 20 g (44 mmol) 2,6-Bis(2,4-dimethylphenyl)-4-phenylpyryliumtetrafluoroborat in 150 ml *n*-Butanol eingesetzt. Der nach der Umsetzung mit PH₃ erhaltene Autoklavenaustrag wurde im Vakuum zur Hälfte eingeengt. Der dabei ausgefallene Feststoff wurde abgesaugt, mit *n*-Butanol gewaschen und anschließend in Toluol gelöst. Die toluolische Lösung wurde dann solange mit Wasser gewaschen, bis die wässrige Phase neutral war. Nach Entfernen des Lösungsmittels und Waschen mit wenig n-Pentan wurde der Rückstand in 250 ml Diethylether/Methanol (3:2) gelöst. Die erhaltene Lösung wurde bei ca. 30 °C im Vakuum solange eingeengt, bis ein Feststoff ausfiel. Der Feststoff wurde abgesaugt, mit wenig Methanol und n-Pentan gewaschen und anschließend im Hochvakuum getrocknet. Ausbeute: 8.9 g (53 %) weißer Feststoff.

### Beispiel 3

### Herstellung von 2,6-Bis(2-methylphenyl)-4-phenylphosphabenzol

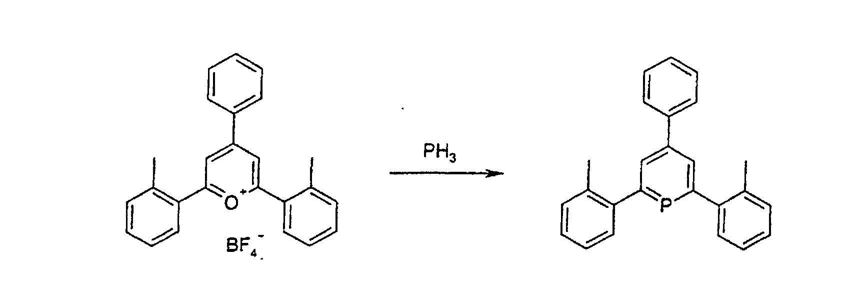

Es wurden zwei mal je 15 g (35 mmol) 2,6-Bis(2-methylphenyl)-4-phenylpyryliumtetrafluoroborat in 150 ml *n*-Butanol eingesetzt. Die beiden nach der Umsetzung mit PH₃ erhaltene Autoklavenausträge wurde vereinigt und bei ca. 80°C im Vakuum bis auf ca. 50 ml eingeengt. Der dabei ausgefallene Feststoff wurde abgesaugt, mit *n*-Pentan gewaschen und anschließend in Toluol gelöst. Die toluolische Lösung wurde dann solange mit Wasser gewaschen, bis die wässrige Phase neutral war. Nach Entfernen des Lösungsmittels wurde der Rückstand in Methanol suspendiert und dann solange mit Dichlormethan versetzt, bis der Feststoff vollständig gelöst war. Die erhaltene Lösung wurde bei ca. 40°C im Vakuum solange eingeengt, bis ein Feststoff ausfiel. Der Feststoff wurde abgesaugt, mit wenig *n*-Pentan gewaschen und anschließend im Hochvakuum getrocknet. Ausbeute: 15.6 g (63 %) weißer Feststoff.

### Beispiel 4

### Herstellung von 2,6-Bis(2,4,5-trimethylphenyl)-4 phenylphosphabenzol

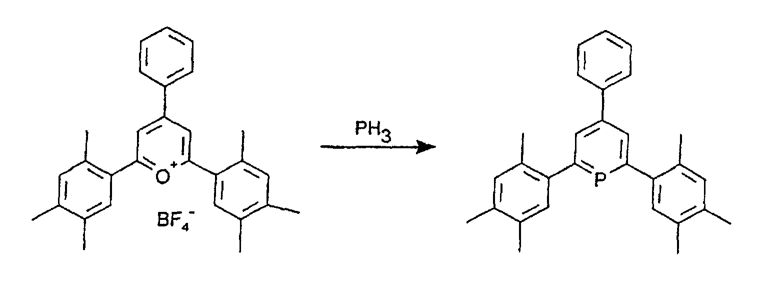

Es wurden 2.1 g (4.4 mmol) 2,6-Bis(2,4,5-trimethylphenyl)-4-phenylpyryliumtetrafluoroborat in 150 ml n-Butanol eingesetzt. Der nach der Umsetzung mit PH₃ erhaltene Autoklavenaustrag wurde,bei ca. 80 °C im Vakuum bis auf ca. 50 ml eingeengt. Der dabei ausgefallene Feststoff wurde abgesaugt, mit *n*-Pentan gewaschen und anschließend in Toluol gelöst. Die toluolische Lösung wurde dann solange mit Wasser gewaschen, bis die wässrige Phase neutral war. Nach Entfernen des Lösungsmittels und Waschen mit wenig *n*-Pentan wurde der Rückstand in Dichlormethan gelöst. Die erhaltene Lösung wurde mit Methanol verdünnt und dann bei 30 °C im Vakuum solange eingeengt, bis ein Feststoff ausfiel. Der Feststoff wurde abgesaugt, mit wenig *n*-Pentan gewaschen und anschließend im Hochvakuum getrocknet. Ausbeute: 0.8 g (45 %) hellgelber Feststoff.

### Beispiel 5

### Herstellung von 10-Phenyl-1,2,7,8-dibenzo-3,4,5,6-tetrahydro-9-phosphaanthracen

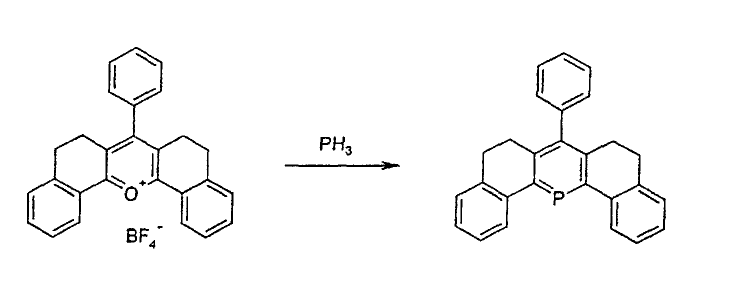

Es wurden 3.5 g (7.8 mmol) 10-Phenyl-1,2,7,8-dibenzo-3,4,5,6-tetrahydro-9-oxoniaanthracentetrafluoroborat in 150 ml Ethanol eingesetzt. Der nach der Umsetzung mit PH₃ erhaltene Autoklavenaustrag wurde bei ca. 80 °C im Vakuum von flüchtigen Bestandteilen befreit. Der orangefarbene Rückstand wurde in ca. 200 ml warmem Toluol suspendiert und sofort über eine Fritte filtriert. Die toluolische Lösung wurde dann solange mit Wasser gewaschen, bis die wässrige Phase neutral war. Nach Entfernen des Lösungsmittels bei 80 °C im Vakuum wurde der verbleibende Feststoff in ca. 30 ml Methanol und ca. 200 ml Dichlormethan gelöst und dann bei 50 °C im Vakuum langsam solange eingeengt, bis ein Feststoff ausfiel. Der Feststoff wurde abgesaugt, mit wenig *n*-Pentan gewaschen und anschließend im Hochvakuum getrocknet. Ausbeute: 0.7 g (24 %) hellgelber Feststoff.

### Allgemeine Versuchsbeschreibung zur Hydroformylierung

Rhodiumprecursor, Ligand und Lösungsmittel wurden unter Stickstoff-Inertgas in einem Schlenkrohr gemischt. Die erhaltene Lösung wurde in einen mit CO/H₂ (1:1) gespülten 100 ml Autoklaven (Material HC) überführt. Es wurden 5 bar CO/H₂ (1:1) kalt aufgepresst. Unter kräftigen Rühren mit einem Begasunsrührer wurde das Reaktionsgemisch innerhalb von 30 min auf die gewünschte Temperatur erhitzt. Über eine Schleuse wurde dann das eingesetzte Olefin mit CO/H2-Überdruck in den Autoklaven gepresst. Darauf wurde sofort mittels CO/H2 (1:1) der gewünschte Reaktionsdruck eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC mit internem Standard und Korrekturfaktor durchgeführt.

### Beispiel 6

### Niederdruck-Hydroformylierung von 1-Octen

Ausgehend von 2.2 mg Rhodiumdicarbonylacetylacetonat (0.009 mmol), 53.9 mg (0.142 mmol) 2,6-Bis(2,4-dimethylphenyl)-4-phenylphosphabenzol aus Beispiel 2, 6.0 g (54 mmol) 1-Octen und 6.0 g Toluol erhielt man bei 90°C, 10 bar CO/H₂ und 4 h gemäß der allgemeinen Versuchsdurchführung einen 1-Octen-Umsatz von 100 %. Die Ausbeute an Nonanalen betrug 91 %, die Selektivität zu n-Nonanal (n-Anteil) betrug 44 % und die Selektivität zu n-Nonanal und 2-Methyloctanal (α-Anteil) betrug 80 %.

### Beispiel 7

### Niederdruck-Hydroformylierung von 1-Octen

Ausgehend von 1.6 mg Rhodiumdicarbonylacetylacetonat (0.006 mmol), 44.5 mg (0.125 mmol) 2,6-Di(2-methylphenyl)-4-phenylphosphabenzol aus Beispiel 3, 5.9 g (53 mmol) 1-Octen und 5.8 g Toluol erhielt man bei 90°C, 10 bar CO/H₂ und 4 h gemäß der allgemeinen Versuchsdurchführung einen 1-Octen-Umsatz von 99 %. Die Ausbeute an Nonanalen betrug 70 %, die Selektivität zun n-Nonanal (n-Anteil) betrug 44 % und die Selektivität zu n-Nonanal und 2-Methyloctanal (α-Anteil) betrug 81 %.

### Beispiel 8

### Niederdruck-Hydroformylierung von 1-Octen

Ausgehend von 1,5 mg Rhodiumdicarbonylacetylacetonat (0.006 mmol), 46.9 mg (0.125 mmol) 10-Phenyl-1,2,7,8-dibenzo-3,4,5,6-tetrahydro-9-phosphaanthracen aus Beispiel 5, 5.9 g (53 mmol) 1-Octen und 6.0 g Toluol erhielt man bei 90°C, 10 bar CO/H₂ und 4 h gemäß der allgemeinen Versuchsdurchführung einen 1-Octen-Umsatz von 57 %. Die Ausbeute an Nonanalen betrug 3 %, die Selektivität zu n-Nonanal (n-Anteil) betrug 73 % und die Selektivität zu n-Nonanal und 2-Methyloctanal (α-Anteil) betrug 100 %.

### Beispiel 9

### Niederdruck-Hydroformylierung von 1-Octen

Ausgehend von 1.7 mg Rhodiumdicarbonylacetylacetonat (0.007 mmol), 52.0 mg (0.127 mmol) 2,6-Bis(2,4,5-trimethylphenyl)-4-phenylphosphabenzol aus Beispiel 4, 5.8 g (52 mmol) 1-Octen und 6.0 g Toluol erhielt man bei 90°C, 10 bar CO/H₂ und 4 h gemäß der allgemeinen Versuchsdurchführung einen 1-Octen-Umsatz von 100 %. Die Ausbeute an Nonanalen betrug 89 %, die Selektivität zu n-Nonanal (n-Anteil) betrug 43 % und die Selektivität zu n-Nonanal und 2-Methyloctanal (α-Anteil) betrug 80 %.

### Beispiel 10

### Mitteldruck-Hydroformylierung von Octen-N2

Ausgehend von 6.5 mg Rhodiumdicarbonylacetylacetonat (0.025 mmol), 201.2 mg (0.493 mmol) 2,6-Bis(2,4,5,-trimethylphenyl)-4-phenylphosphabenzol aus Beispiel 4, 23.4 g (209 mmol) Octen-N2 und 23.7 g Texanol® erhielt man bei 100°C und 60 bar CO/H₂ gemäß der allgemeinen Versuchsdurchführung einen Octen-N2-Umsatz von 76 % nach 4 h Reaktionszeit und 93 % nach 24 h Reaktionszeit. Die Ausbeute an Nonanale betrug 76 % nach 4 h Reaktionszeit und 93 % nach 24 h Reaktionszeit.

### Allgemeine Versuchsbeschreibung zur Untersuchung des Abbauverhaltens von Phosphabenzolen unter Hydroformylierungsbedingungen

Rhodiumprecursor, Ligand, 1-Octen und Nonanal wurden unter Stickstoff-Inertgas in einem Schlenkrohr gemischt. Die erhaltene Lösung wurde in einen mit CO/H₂ (1:1) gespülten 100 ml Autoklaven (Material HC) überführt. Es wurden 10 bar CO/H₂ (1:1) kalt aufgepresst. Unter kräftigem Rühren mit einem Begasungsrührer wurde das Reaktionsgemisch innerhalb von 30 min auf 150°C erhitzt. Dann wurde mittels CO/H₂ (1:1) ein Reaktionsdruck von 60 bar eingestellt. Während der Reaktion wurde der Druck im Reaktor durch Nachpressen über einen Druckregler auf Druckniveau gehalten. Nach 3 d Reaktionszeit wurde der Autoklav abgekühlt, entspannt und unter Inertgas entleert. Die Quantifizierung des Phosphabenzolabbaus erfolgte mittels GC mit einem NP-spezifischen Detektor und in ausgewählten Fällen zusätzlich durch quantifizierende ³¹P-NMR-Spektroskopie.

### Beispiel 11 (Vergleich)

### Abbauverhalten von 2,4,6-Triphenylphosphabenzol

Ausgehend von 7.3 mg Rhodiumdicarbonylacetylacetonat (0.028 mmol), 160.0 mg (0.494 mmol) 2,4,6-Triphenylphosphabenzol, 12.0 g (107 mmol) 1-Octen und 12.0 g (85 mmol) Isononanal erhielt man gemäß der allgemeinen Versuchsdurchführung einen Phosphabenzolabbau von 70 % (GC) und 80 % (³¹P-NMR-Spektroskopie).

### Beispiel 12 (Vergleich)

### Abbauverhalten von 2,3,5,6-Tetraphenylphosphabenzol

Ausgehend von *5.2* mg Rhodiumdicarbonylacetylacetonat (0.020 mmol), 118.0 mg (0.295 mmol) 2,3,5,6-Tetraphenylphosphabenzol, 8.6 g (77 mmol) 1-Octen und 8.6 g (61 mmol) Isononanal erhielt man gemäß der allgemeinen Versuchsdurchführung einen Phosphabenzolabbau von 47 % (GC).

### Beispiel 13 (Vergleich)

### Abbauverhalten von 2,3,4,5,6-Pentaphenylphosphabenzol

Ausgehend von 1.3 mg Rhodiumdicarbonylacetylacetonat (0.005 mmol), 37.0 mg (0.078 mmol) 2,3,4,5,6-Pentaphenylphosphabenzol, 6.0 g (54 mmol) 1-Octen und 6.0 g (43 mmol) Isononanal erhielt man gemäß der allgemeinen Versuchsdurchführung einen Phosphabenzolabbau von 60 % (GC).

### Beispiel 14 (Vergleich)

### Abbauverhalten von 2,6-Bis(2-naphthyl)-4-phenylphosphabenzol

Ausgehend von 8.1 mg Rhodiumdicarbonylacetylacetonat (0.031 mmol), 153.3 mg (0.361 mmol) 2,6-Bis(2-naphthyl)-4-phenylphosphabenzol, 14.0 g (125 mmol) 1-OCten und 14.0 g (98 mmol) Isononanal erhielt man gemäß der allgemeinen Versuchsdurchführung einen Phosphabenzolabbau von 54 % (GC).

### Beispiel 15

### Abbauverhalten von 2,6-Bis(2,4-dimethylphenyl)-4-phenylphosphabenzol

Ausgehend von 7.3 mg Rhodiumdicarbonylacetylacetonat (0.028 mmol), 167.1 mg (0.439 mmol) 2,6-Bis(2,4-dimethylphenyl)-4-phenylphosphabenzol, 12.0 g (107 mmol) 1-Octen und 12.0 g (84 mmol) Isononanal erhielt man gemäß der allgemeinen Versuchsdurchführung einen Phosphabenzolabbau von 5 % (GC).

### Beispiel 16

### Abbauverhalten von 2,6-Di(2-methylphenyl)-4-phenylphosphabenzol

Ausgehend von 7.3 mg Rhodiumdicarbonylacetylacetonat (0.028 mmol), 165.0 mg (0.460 mmol) 2,6-Di(2-methylphenyl)-4-phenylphosphabenzol, 12.0 g (107 mmol) 1-Octen und 12.0 g (84 mmol) Isononanal erhielt man gemäß der allgemeinen Versuchsdurchführung einen Phosphabenzolabbau von 14 % (GC).

### Beispiel 17

### Abbauverhalten von 2,6-Bis(2,4,5-trimethylphenyl)-4-phenylphosphabenzol

Ausgehend von 3.1 mg Rhodiumdicarbonylacetylacetonat (0.012 mmol), 94.8 mg (0.232 mmol) 2,6-Bis(2,4,5-trimethylphenyl)-4-phenylphosphabenzol, 6.0 g (54 mmol) 1-Octen und 6.0 g (42 mmol) Isononanal erhielt man gemäß der allgemeinen Versuchsdurchführung einen Phosphabenzolabbau von 19 % (GC) und 40 % (³¹P-NMR-Spektroskopie).

## Patentansprüche

1. Phosphabenzolverbindungen der allgemeinen Formel (I) 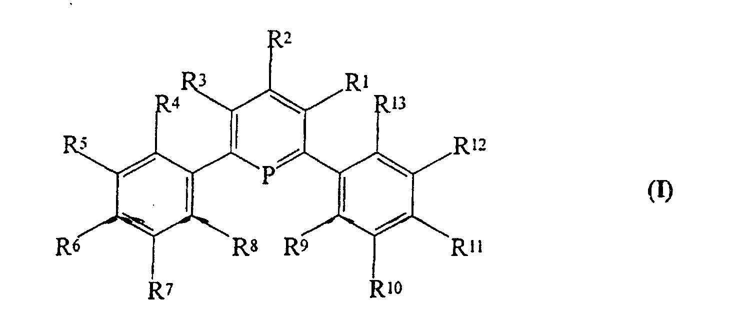 in der die Reste R¹ bis R¹³ unabhängig voneinander Wasserstoff, COOM, SO₃M, NR₃X, NR₂, OR, COOR oder SR (mit M = Wasserstoff, NH₄ oder Alkalimetall, X = Anion, R = Wasserstoff oder C₁₋₆-Alkyl), oder C₁₋₁₂-Alkyl, C₆₋₁₂-Aryl, C₇₋₁₂-Aralkyl, C₇₋₁₂-Alkaryl oder C₃₋₆-Heteroaromaten bedeuten, wobei die Alkyl-, Aryl-, Alkaryl- und Aralkylreste mit den davor genannten Resten substituiert sein können und zwei oder mehrere der Reste zu aliphatischen oder annelierten Ringen verbunden sein können, wobei mindestens einer der Reste R⁴ und R⁸ und mindestens einer der Reste R⁹ und R¹³ von Wasserstoff verschieden sind.

2. Phosphabenzolverbindungen nach Anspruch 1, **dadurch gekennzeichnet, daß** mindestens einer der Reste R⁴ und R⁸ und mindestens einer der Reste R⁹ und R¹³ unabhängig voneinander C₁₋₁₂-Alkyl, C₆₋₁₂-Aryl, C₇₋₁₂-Aralkyl oder C₇₋₁₂-Alkaryl bedeuten, oder (R⁴ und R³) und/oder (R¹³ und R¹) einen C₂₋₄-Alkylenrest bilden.

3. Phosphabenzolverbindungen nach Anspruch 2, **dadurch gekennzeichnet, daß** mindestens einer der Reste R⁴ und R⁸ und mindestens einer der Reste R⁹ und R¹³ unabhängig voneinander C₁₋₆-Alkyl bedeuten oder (R⁴ und R³) und (R¹³ und R¹) jeweils einen C₂₋₃-Alkylenrest bilden.

4. Phosphabenzolverbindungen nach einem der Ansprüche 1 bis 3, **dadurch gekennzeichnet, daß** der Rest R² einen Phenylrest bedeutet, der durch 1 bis 5 C₁₋₆-Alkylreste substituiert sein kann.

5. Phosphabenzolverbindungen nach einem der Ansprüche 1 bis 4, **dadurch gekennzeichnet , daß** die Reste R¹ und R³ Wasserstoff bedeuten und jeweils maximal 3 der Reste R⁴ bis R⁸ und R⁹ bis R¹³ von Wasserstoff verschieden sind.

6. Verfahren zur Herstellung von Verbindungen der allgemeinen Formel (I) gemäß einem der Ansprüche 1 bis 5 durch Umsetzung entsprechender Pyryliumsalze mit PH₃ in Gegenwart oder Abwesenheit einer katalytischen Menge Säure oder Base und in Anwesenheit oder Abwesenheit eines Löseoder Verdünnungsmittels.

7. Verfahren nach Anspruch 6, **dadurch gekennzeichnet, daß** die Pyryliumsalze mit PH₃ bei einer Temperatur oberhalb von 0°C zusammengebracht und bei einer Temperatur oberhalb von 0°C bis 200°C und einem Druck oberhalb von 1 bar umgesetzt werden, wobei die Umsetzung bei einem PH₃-Partialdruck im Bereich von 0,1 bis 100 bar erfolgt.

8. Verwendung der Verbindungen gemäß einem der Ansprüche 1 bis 5 zur Herstellung von Komplexen mit Metallen der VIII. Nebengruppe des Periodensystems der Elemente.

9. Komplexe von Verbindungen gemäß einem der Ansprüche 1 bis 5 mit Metallen der VIII. Nebengruppe des Periodensystems der Elemente.

10. Verwendung von Komplexen gemäß Anspruch 9 als Katalysator zur Hydroformylierung von Olefinen mit CO/H₂.

11. Verfahren zur Hydroformylierung von C₂₋₂₀-Olefinen durch Umsetzung mit CO und H₂ in Gegenwart von Komplexen gemäß Anspruch 9 als Katalysator bei einer Temperatur im Bereich von 0 bis 200°C und einem Druck im Bereich von 1 bis 700 bar.

## Claims

1. A phosphabenzene compound of the formula (I) 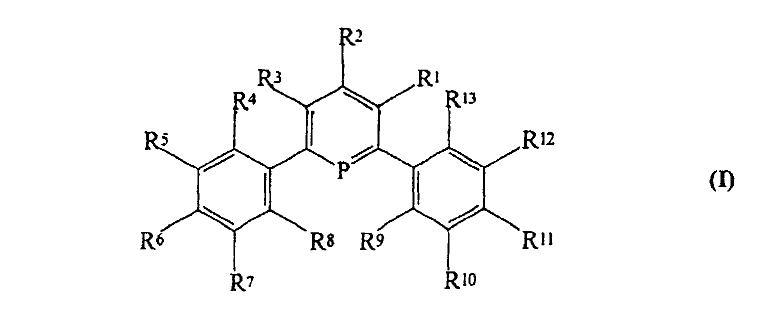 where the radicals R¹ to R¹³ are, independently of one another, hydrogen, COOM, SO₃M, NR₃X, NR₂, OR, COOR or SR (where M = hydrogen, NH₄ or alkali metal, X = anion, R = hydrogen or C₁-C₆-alkyI), or C₁-C₁₂-alkyl, C₆-C₁₂-aryl, C₇-C₁₂-aralkyl, C₇-C₁₂-alkaryl or C₃-C₆-heteroaromatics, where the alkyl, aryl, alkaryl and aralkyl radicals may bear the abovementioned radicals as substituents and two or more of the radicals may be joined to form aliphatic or fused-on rings, where at least one of the radicals R⁴ and R⁸ and at least one of the radicals R⁹ and R¹³ is not hydrogen.

2. A phosphabenzene compound as claimed in claim 1, wherein at least one of the radicals R⁴ and R⁸ and at least one of the radicals R⁹ and R¹³ are, independently of one another, C₁-C₁₂-alkyI, C₆-C₁₂-aryl, C₇-C₁₂-aralkyl or C₇-C₁₂-alkaryl, or (R⁴ and R³) and/or (R¹³ and R¹) form a C₂-C₄-alkylene radical.

3. A phosphabenzene compound as claimed in claim 2, wherein at least one of the radicals R⁴ and R⁸ and at least one of the radicals R⁹ and R¹³ are, independently of one another, C₁-C₆-alkyI, or (R⁴ and R³) and (R¹³ and R¹) in each case form a C₂-C₃-alkylene radical.

4. A phosphabenzene compound as claimed in any of claims 1 to 3, wherein the radical R² is a phenyl radical which may be substituted by from 1 to 5 C₁-C₆-alkyl radicals.

5. A phosphabenzene compound as claimed in any of claims 1 to 4, wherein the radicals R¹ and R³ are hydrogen and in each case not more than 3 of the radicals R⁴ to R⁸ and R⁹ to R¹³ are not hydrogen.

6. A process for preparing a compound of the formula (I) as claimed in any of claims 1 to 5 by reacting a corresponding pyrylium salt with PH₃ in the presence or absence of a catalytic amount of acid or base and in the presence or absence of a solvent or diluent.

7. A process as claimed in claim 6, wherein the pyrylium salt is brought into contact with PH₃ at above 0°C and reacted at above 0°C-200°C and a pressure above 1 bar, with the reaction being carried out at a PH₃ partial pressure in the range from 0.1 to 100 bar.

8. The use of a compound as claimed in any of claims 1 to 5 for preparing complexes of metals of transition group VIII of the Periodic Table of the Elements.

9. A complex of a compound as claimed in any of claims 1 to 5 with a metal of transition group VIII of the Periodic Table of the Elements.

10. The use of a complex as claimed in claim 9 as catalyst for the hydroformylation of olefins using CO/H₂.

11. A process for the hydroformylation of C₂-C₂₀-olefins by reaction with CO and H₂ in the presence of a complex as claimed in claim 9 as catalyst at from 0 to 200°C and a pressure in the range from 1 to 700 bar.

## Revendications

1. Phosphabenzènes de formule générale (I) 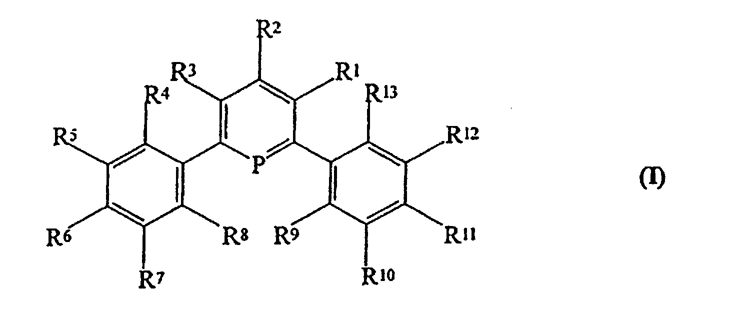 dans laquelle les radicaux R¹ à R¹³ représentent chacun indépendamment de l'autre un atome d'hydrogène, COOM, SO₃M, NR₃X, NR₂, OR, COOR ou SR (M étant un atome d'hydrogène, NH₄ ou un métal alcalin, X étant un anion, R étant un atome d'hydrogène ou un groupe alkyle en C₁₋₆) ou un groupe alkyle en C₁₋₁₂, aryle en C₆₋₁₂, aralkyle en C₇₋₁₂, alcaryle en C₇₋₁₂ ou un radical hétéroaromatique en C₃₋₆, les résidus alkyle, aryle, alcaryle et aralkyle pouvant être substitués par les radicaux mentionnés ci-dessus, et au moins deux des radicaux pouvant être liés en donnant des noyaux aliphatiques ou condensés, au moins l'un des radicaux R⁴ et R⁸ et au moins l'un des radicaux R⁹ et R¹³ étant différents de l'hydrogène.

2. Phosphabenzènes selon la revendication 1, **caractérisés en ce qu'**au moins l'un des radicaux R⁴ et R⁸ et au moins l'un des radicaux R⁹ et R¹³ représente indépendamment de l'autre un groupe alkyle en C₁₋₁₂, aryle en C₆₋₁₂, aralkyle en C₇₋₁₂ ou alcaryle en C₇₋₁₂, ou encore (R⁴ et R³) et/ou (R¹³ et R¹) forment un radical alkylène en C₂₋₄.

3. Phosphabenzènes selon la revendication 2, **caractérisés en ce qu'**au moins l'un des radicaux R⁴ et R⁸ et au moins l'un des radicaux R⁹ et R¹³ représente indépendamment de l'autre un groupe alkyle en C₁₋₆, ou encore (R⁴ et R³) et (R¹³ et R¹) forment chacun un radical alkylène en C₂₋₃.

4. Phosphabenzènes selon l'une des revendications 1 à 3, **caractérisés en ce que** le radical R² est un groupe phényle pouvant être substitué par 1 à 5 résidus alkyle en C₁₋₆.

5. Phosphabenzènes selon l'une des revendications 1 à 4, **caractérisés en ce que** les radicaux R¹ et R³ sont des atomes d'hydrogène, et au maximum 3 des radicaux R⁴ à R⁸ et au maximum trois des radicaux R⁹ à R¹³ sont différents de l'hydrogène.

6. Procédé de préparation de composés de formule générale (I) selon l'une des revendications 1 à 5 par réaction des sels de pyrylium correspondants avec du PH₃ en présence ou en l'absence d'une quantité catalytique d'un acide ou d'une base et en présence ou en l'absence d'un solvant ou d'un diluant.

7. Procédé selon la revendication 6, **caractérisé en ce que** les sels de pyrylium sont réunis au PH₃ à une température supérieure à 0°C et sont mis à réagir à une température supérieure à 0°C et allant jusqu'à 200°C et sous une pression supérieure à 1 bar, la réaction ayant lieu sous une pression partielle de PH₃ comprise entre 0,1 et 100 bar.

8. Utilisation des composés selon l'une des revendications 1 à 5 pour préparer des complexes avec des métaux du VIIIème groupe secondaire du Tableau Périodique des Eléments.

9. Complexes de composés selon l'une des revendications 1 à 5 avec des métaux du VIIIème groupe secondaire du Tableau Périodique des Eléments.

10. Utilisation de complexes selon la revendication 9, en tant que catalyseur pour l'hydroformylation d'oléfines avec du CO/H₂.

11. Procédé d'hydroformylation d'oléfines en C₂₋₂₀ par réaction avec du CO et du H₂ en présence de complexes selon la revendication 9 en tant que catalyseur à une température comprise entre 0 et 200°C et sous une pression de 1 à 700 bar.
